(19) **Europäisches Patentamt / European Patent Office / Office européen des brevets**

(11) **EP 4 685 685 A1**

(12) **EUROPEAN PATENT APPLICATION**
published in accordance with Art. 153(4) EPC

(43) Date of publication:
**28.01.2026 Bulletin 2026/05**

(21) Application number: **24773912.1**

(22) Date of filing: **05.03.2024**

(51) International Patent Classification (IPC):
**G06F 30/20** (2020.01)  **G21C 17/00** (2006.01)

(52) Cooperative Patent Classification (CPC):
Y02E 30/30

(86) International application number:
**PCT/CN2024/080150**

(87) International publication number:
**WO 2024/193344 (26.09.2024 Gazette 2024/39)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **22.03.2023  CN 202310283228**

(71) Applicants:
• **China Nuclear Power Technology Research Institute Co., Ltd.**
**Shenzhen, Guangdong 518031 (CN)**
• **China General Nuclear Power Corporation**
**Shenzhen, Guangdong 518028 (CN)**
• **CGN Power Co., Ltd.**
**Shenzen, Guangdong 518028 (CN)**

(72) Inventors:
• **WANG, Xin**
**Shenzhen, Guangdong 518031 (CN)**

• **ZHU, Yuxiang**
**Shenzhen, Guangdong 518031 (CN)**
• **XU, Huaijin**
**Shenzhen, Guangdong 518031 (CN)**
• **ZHAO, Changyou**
**Shenzhen, Guangdong 518031 (CN)**
• **LI, Zhifeng**
**Shenzhen, Guangdong 518031 (CN)**
• **ZHAO, Yan**
**Shenzhen, Guangdong 518031 (CN)**
• **YU, Chao**
**Shenzhen, Guangdong 518031 (CN)**
• **LIN, Jun**
**Shenzhen, Guangdong 518031 (CN)**
• **HE, Mingtao**
**Shenzhen, Guangdong 518031 (CN)**
• **LU, Xianghui**
**Shenzhen, Guangdong 518031 (CN)**

(74) Representative: **Greaves Brewster LLP**
**Copa House**
**Station Road**
**Cheddar, Somerset BS27 3AH (GB)**

(54) **METHOD AND APPARATUS FOR QUANTIFYING INDICATION SIGNALS OF NUCLEAR INSTRUMENT SYSTEM, AND DEVICE, AND STORAGE MEDIUM**

(57)    The present application relates to a method and apparatus for quantifying indication signals of a nuclear instrument system, and a device, and a storage medium. The method comprises: simulating the power distribution of a reactor core under various operating conditions, so as to obtain power distribution parameters of the reactor core under the various operating conditions; and according to the power distribution parameters of the reactor core under the different operating conditions and according to corresponding benchmark power distribution parameters, quantifying indication signals of the reactor core.

The use of the present method allows for quantifying the uncertainty of indication signals of the reactor core by means of theoretical analysis, allowing for obtaining more-accurate indication signals; moreover, the present solution does not require the collection of actual measurement data under actual operating conditions, and is suited to controlling novel reactor cores lacking actual measurement data and in designing constant values of protection signals, and therefore the present solution is highly applicable.

EP 4 685 685 A1

simulating power distributions of a reactor core under different operating conditions to obtain power distribution parameters of the reactor core under the different operating conditions — S201

quantifying the indication signal of an ex-core detector based on a non-parametric statistical analysis method according to the power distribution parameters of the reactor core under the different operating conditions and corresponding reference power distribution parameters — S202

FIG. 2

**Description**

CROSS-REFERENCE TO RELATED APPLICATIONS

This application claims priority to Chinese patent application No. 2023102832285, filed with China National Intellectual Property Administration on March 22, 2023, entitled "METHOD AND APPARATUS FOR QUANTIFYING INDICATION SIGNAL OF NUCLEAR INSTRUMENT SYSTEM, DEVICE AND STORAGE MEDIUM", the entire text of which is hereby incorporated by reference.

TECHNICAL FIELD

The present application relates to the field of nuclear instrument measurement technology, and in particular to a method and an apparatus for quantifying an indication signal of a nuclear instrument system, a device, and a storage medium.

BACKGROUND

The nuclear instrument system (also named a nuclear reactor protection system, RPN), is a key measurement system in nuclear power plants, and the main measurement principle thereof is that there is a linear relationship between a reactor core nuclear power, a neutron flux rate leaking to an ex-core detector, and a response of an ex-core detector. However, during a unit operation, the relationship between the reactor core nuclear power from the RPN and the response of the ex-core detector is not strictly linear, which will lead to a deviation between an indication signal of the RPN and an actual signal, that is, there is uncertainty in the indication signal. In order to reduce the uncertainty of the indication signal, it is necessary to evaluate the uncertainty of the indication signal.

Currently, the uncertainty of the indication signal of the RPN is mainly evaluated based on the operation data statistics for the RPN during a long-term operation, or the uncertainty of the indication signal of the RPN is evaluated based on the experience of the inspection or maintenance personnel.

However, the above evaluation methods have the problem of low accuracy.

SUMMARY

In view of the above technical problem, it is necessary to provide a method and an apparatus for quantifying an indication signal of a nuclear instrument system, a device and a storage medium that can improve the accuracy of the evaluation of the indication signal of the nuclear instrument system.

In a first aspect, the present application provides a method for quantifying an indication signal of a nuclear instrument system. The method comprises:

simulating power distributions of a reactor core under different operating conditions to obtain power distribution parameters of the reactor core under the different operating conditions; and

quantifying the indication signal of an ex-core detector based on a non-parametric statistical analysis method according to the power distribution parameters of the reactor core under the different operating conditions and corresponding reference power distribution parameters.

In one of the embodiments, simulating the power distributions of the reactor core under the different operating conditions to obtain the power distribution parameters of the reactor core under the different operating conditions comprises:

constructing an actual physical model of the reactor core;

obtaining characteristic parameters under the different operating conditions; and

inputting the characteristic parameters under the different operating conditions into the actual physical model of the reactor core to obtain the power distribution parameters of the reactor core under the different operating conditions.

In one of the embodiments, the different operating conditions comprise normal operating conditions and accident operating conditions, and simulating the power distributions of the reactor core under the different operating conditions to obtain the power distribution parameters of the reactor core under the different operating conditions comprises:

simulating the power distributions of the reactor core under the normal operating conditions and the power distributions of the reactor core under the accident operating conditions to obtain the power distribution parameters of the reactor core under the normal operating conditions and the power distribution parameters of the reactor core under the accident operating conditions.

In one of the embodiments, quantifying the indication signal of the ex-core detector based on the non-parametric statistical analysis method according to the power distribution parameters of the reactor core under the different operating conditions and the corresponding reference power distribution parameters comprises:

determining the indication signal of the ex-core detector under each of the different operating conditions according to the power distribution parameters of the reactor core under the different operating conditions; and quantifying the indication signal of the ex-core detector based on the non-parametric statistical analysis method according to the indication signal of the ex-core detector under each of the different operating conditions and the reference power distribution parameters.

In one of the embodiments, the different operating conditions comprise normal operating conditions and accident operating conditions, and determining the indication signal of the ex-core detector under each of the different operating conditions according to the power distribution parameters of the reactor core under the different operating conditions comprises:

determining detection currents of the ex-core detector under the normal operating conditions and under the accident operating conditions based on the power distribution parameters of the reactor core under the normal operating conditions and the power distribution parameters under the accident operating conditions; determining calibration parameters according to power distribution parameters of the reactor core during a calibration process; and determining the indication signal of the ex-core detector under each of the normal operating conditions and the accident operating conditions based on the calibration parameters and the detection current of the ex-core detector under each of the normal operating conditions and the accident operating conditions.

In one of the embodiments, determining the detection currents of the ex-core detector under the normal operating conditions and under the accident operating conditions based on the power distribution parameters of the reactor core under the normal operating conditions and the power distribution parameters under the accident operating conditions comprises:

obtaining responses of the ex-core detector; and determining the detection currents of the ex-core detector under the normal operating conditions and under the accident operating conditions based on the power distribution parameters of the reactor core under the normal operating conditions, the power distribution parameters under the accident operating conditions, and the responses of the ex-core detector.

In one of the embodiments, determining the calibration parameters according to the power distribution parameters of the reactor core during the calibration process comprises:

obtaining a response of the ex-core detector and a reference power distribution parameter of the reactor core during the calibration process; determining a detection current of the reactor core during the calibration process based on the reference power distribution parameter of the reactor core during the calibration process and the response of the ex-core detector; and determining the calibration parameters according to the detection current of the reactor core during the calibration process and a reference physical parameter of the reactor core during the calibration process.

In a second aspect, the present application further provides an apparatus for quantifying an indication signal of a nuclear instrument system. The apparatus comprises:

an acquisition module, configured to simulate power distributions of a reactor core under different operating conditions to obtain power distribution parameters of the reactor core under the different operating conditions; and a quantization module, configured to quantify the indication signal of an ex-core detector based on a non-parametric statistical analysis method according to the power distribution parameters of the reactor core under the different operating conditions and corresponding reference power distribution parameters.

In a third aspect, the present application further provides a computer device. The computer device includes a memory and a processor. The memory has a computer program stored thereon, and the processor, when executing the computer program, performs the following steps:

simulating power distributions of a reactor core under different operating conditions to obtain power distribution parameters of the reactor core under the different operating conditions; and

quantifying the indication signal of an ex-core detector based on a non-parametric statistical analysis method according to the power distribution parameters of the reactor core under the different operating conditions and corresponding reference power distribution parameters.

In a fourth aspect, the present application further provides a computer-readable storage medium, having a computer program stored thereon. The computer program, when executed by a processor, performs the following steps:

simulating power distributions of a reactor core under different operating conditions to obtain power distribution parameters of the reactor core under the different operating conditions; and

quantifying the indication signal of an ex-core detector based on a non-parametric statistical analysis method according to the power distribution parameters of the reactor core under the different operating conditions and corresponding reference power distribution parameters.

In a fifth aspect, the present application further provides a computer program product. The computer program product includes a computer program. The computer program, when executed by a processor, causes the processor to perform the following steps:

simulating power distributions of a reactor core under different operating conditions to obtain power distribution parameters of the reactor core under the different operating conditions; and

quantifying the indication signal of an ex-core detector based on a non-parametric statistical analysis method according to the power distribution parameters of the reactor core under the different operating conditions and corresponding reference power distribution parameters.

In the method and apparatus for quantifying the indication signal of the nuclear instrument system, the device, and the storage medium, the power distributions of the reactor core under different operating conditions are simulated to obtain the power distribution parameters of the reactor core under the different operating conditions, and the indication signal of the ex-core detector is quantified based on the non-parametric statistical analysis method according to the power distribution parameters of the reactor core under different operating conditions and the corresponding reference power distribution parameters. This method considers the power distribution parameters of the reactor core under different operating conditions, and quantifies the indication signal of the ex-core detector based on the non-parametric statistical analysis method according to the power distribution parameters of the reactor core under different operating conditions and the reference power distribution parameters. Compared with the prior art, this solution does not require the collection of measured data under actual operating conditions and is suitable for designing control and protection signal setpoints for a new reactor core where actual measurement data is scarce. Therefore, this solution has strong applicability. In addition, this solution quantifies the uncertainty of the indication signal of the ex-core detector through theoretical analysis, thereby obtaining a more accurate indication signal.

DESCRIPTION OF THE DRAWINGS

In order to better describe and illustrate the embodiments and/or examples of the applications disclosed herein, reference may be made to one or more of the accompanying drawings. Description of the additional details or examples of the accompanying drawings should not be considered to limit the scope of any of the disclosed applications, the presently described embodiments and/or examples, and the best modes currently understood for these applications.

FIG. 1 is a view illustrating an application scenario of a method for quantifying an indication signal of a nuclear instrument system according to an embodiment;

FIG. 2 is a schematic flow chart of a method for quantifying the indication signal of the nuclear instrument system according to an embodiment;

FIG. 3 is a schematic flow chart of step S201 in the embodiment corresponding to FIG. 2;

FIG. 4 is a schematic flow chart of step S202 in the embodiment corresponding to FIG. 2;

FIG. 5 is a schematic flow chart of step S401 in the embodiment corresponding to FIG. 4;

FIG. 6 is a schematic flow chart of step S4011 in the embodiment corresponding to FIG. 5;

FIG. 7 is a schematic flow chart of step S4012 in the embodiment corresponding to FIG. 6;

FIG. 8 is a schematic flow chart of a method for quantifying a signal according to another embodiment;

FIG. 9 is a block diagram showing a structure of an apparatus for quantifying an indication signal of a nuclear instrument system according to an embodiment;

FIG. 10 is a block diagram showing a structure of the apparatus for quantifying the indication signal of the nuclear instrument system according to another embodiment;

FIG. 11 is a block diagram showing a structure of the apparatus for quantifying the indication signal of the nuclear instrument system according to another embodiment.

DETAILED DESCRIPTION OF THE EMBODIMENTS

In order to make the objectives, technical solutions, and advantages of the present application clearer and better understood, the present application is further described in detail below in conjunction with the accompanying drawings and embodiments. It should be understood that the specific embodiments described herein are only used to explain the present application but not intended to limit the present application.

The nuclear instrument system (also named a nuclear reactor protection system, RPN) is a key measurement system in the nuclear power plants, and the main measurement principle thereof is that there is a linear relationship between a reactor core nuclear thermal power, a neutron flux rate leaking to an ex-core detector, and an ex-core detector response. However, during a unit operation, the relationship between the reactor core nuclear power of the RPN and the response of the ex-core detector is not exactly linear, which will lead to a deviation between an indication signal of the RPN and an actual signal, that is, there is uncertainty in the indication signal.

In addition, due to the unique physical design of the reactor core and the calibration method of the ex-core detector, the actual ex-core detector response will be affected by the power distribution. During the operation of the unit, factors such as burnup effects, xenon oscillations and changes in control rod positions will cause the power distribution within the reactor to change. This change will make the relationship between the response of the ex-core detector and the reactor core indication power not strictly linear, which will also lead to a deviation between an indication signal of the RPN and an actual signal of the reactor core.

The indication signal of the RPN is usually used for the protection and control of the reactor core. If the deviation between the indication signal of the RPN and the actual signal is not properly assessed, it may cause the unit to erroneously trigger a control signal and protection signal thus affecting the availability of the unit, or it may cause insufficient conservatism in an accident analysis consideration, thus a corresponding protection cannot be triggered in time in the event of an accident.

Currently, the uncertainty of the indication signal of the RPN is mainly evaluated based on the operation data statistics for the RPN during a long-term operation, or the uncertainty of the indication signal of the RPN is evaluated based on the experience of the inspection or maintenance personnel. However, the accuracy of these methods for evaluating the uncertainty of the indication signal of the RPN is relatively low. This application aims at addressing this problem.

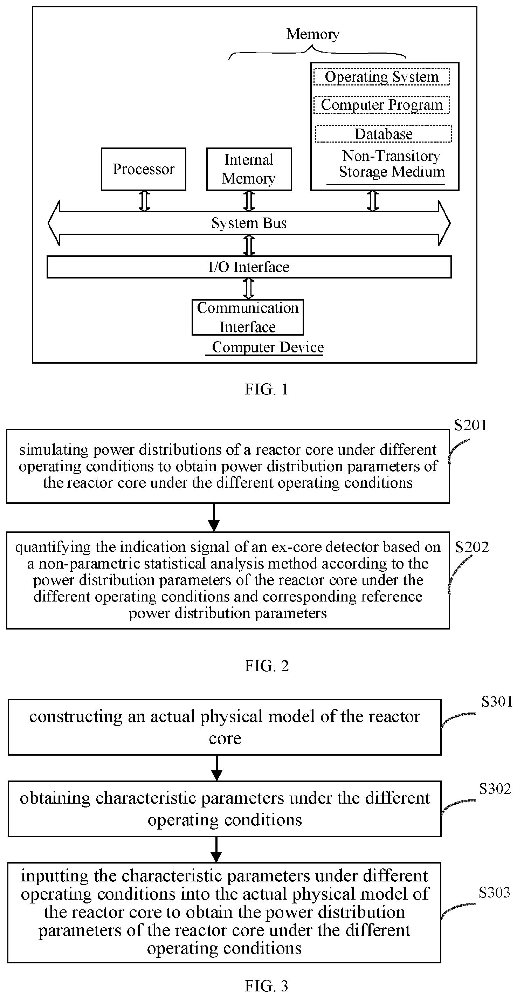

Following the above introduction to the background technology of the method for quantifying the indication signal of the nuclear instrument system provided in the embodiments of the present application, the implementation scenario involved in the method for quantifying the indication signal of the nuclear instrument system provided in the embodiments of the present application will be briefly described hereinafter. The method for quantifying the indication signal of the nuclear instrument system provided in the embodiments of the present application can be applied to a computer device shown in FIG. 1. The computer device includes a processor and a memory connected via a system bus. The memory stores a computer program. The processor, when executing the computer program, can perform the steps of the following method embodiments. Optionally, the computer device may also include an input/output interface. The processor of the computer device is used to provide computing and control capabilities. The memory of the computer device includes a non-transitory storage medium and an internal memory. The non-transitory storage medium stores an operating system, a computer program, and a database. The internal memory provides an environment for the operation of the operating system and computer program in the non-transitory storage medium. The input/output interface of the computer device is used to communicate with external terminals via a network connection. Optionally, the computer device can be a server, a personal computer, a personal digital assistant, or any other terminal device such as a tablet computer, or a mobile phone, etc., or a cloud or remote server, and the computer device is not specifically limited in the embodiments of the present application.

Those skilled in the art will understand that the structure shown in FIG. 1 is merely a block diagram of a portion of the structure related to the solutions of the present application, and does not constitute a limitation on the computer device to which the solutions of the present application is applied. The computer device may specifically include more or fewer components than shown in the figure, or combine certain components, or have a different arrangement of components.

After describing the application scenario of the method for quantifying the indication signal of the nuclear instrument system provided in the embodiments of the present application, a process of quantifying the signal is described hereinafter.

In an embodiment, as shown in FIG. 2, a method for quantifying an indication signal of a nuclear instrument system is provided. The method is described by taking the method applied to the computer device shown in FIG. 1 as an example, and includes the following steps.

At S201, power distributions of a reactor core under different operating conditions are simulated to obtain power distribution parameters of the reactor core under the different operating conditions.

The different operating conditions may include, but are not limited to, normal operating conditions, accident operating conditions, and ideal operating conditions. The power distributions under different operating conditions may include, but are not limited to, a perturbed loading pattern, a burnup, a control rod position, a xenon oscillation time, etc. The power distributions under different operating conditions are different.

A physical reactor core model is a high-fidelity steady-state calculation program developed for numerical reactors. The power distribution parameters vary under different operating conditions.

In an embodiment of the present application, a physical reactor core model for experimental usage can be pre-designed, and the physical reactor core model can operate under different operating conditions to analyze the power distributions of the physical reactor core model under different operating conditions, thereby obtaining the power distribution parameters of the physical reactor core model under different operating conditions. Optionally, a reactor core simulation model can be pre-constructed in a simulation application, and a reactor core simulation model can operate under different operating conditions to analyze the power distributions of the reactor core simulation model under different operating conditions, thereby obtaining the power distribution parameters of the reactor core simulation model under different operating conditions.

When evaluating the uncertainty of the indication signal of the nuclear instrument system, the computer device can analyze the power distributions of the reactor core under different operating conditions using the physical reactor core model or the reactor core simulation model to obtain the power distribution parameters of the reactor core under different operating conditions. It should be noted that both the physical reactor core model and the reactor core simulation model are equivalent models of the actual reactor core and can characterize an actual reactor core state.

At S202, the indication signal of an ex-core detector is quantified based on a non-parametric statistical analysis method according to the power distribution parameters of the reactor core under the different operating conditions and corresponding reference power distribution parameters.

The reference power distribution parameters may be standard power distribution parameters of the reactor core under normal operating conditions and standard power distribution parameters under accident operating conditions, which may be preset in the computer device. The indication signal of the ex-core detector may include an axial power deviation parameter, a nuclear thermal power, etc.

In the embodiment of the present application, after step S201 of obtaining the power distribution parameters of the reactor core under different operating conditions, the computer device can compare the power distribution parameters of the reactor core under different operating conditions with corresponding reference power distribution parameters preset in the computer device to obtain a comparison result. The comparison result can reflect a degree of proximity between the power distribution parameters of the reactor core under different operating conditions and the corresponding reference power distribution parameters preset in the computer device. Exemplarily, if the power distribution parameters of the reactor core under different operating conditions are relatively close to the corresponding reference power distribution parameters, it indicates that the indication signal of the ex-core detector is consistent with the actual signal under the current operating conditions. If the power distribution parameters of the reactor core under different operating conditions are significantly different from the corresponding reference power distribution parameters, it indicates that the indication signal of the ex-core detector is inconsistent with the actual signal under the current operating conditions. Therefore, the computer device can determine the degree of the uncertainty between the power distribution parameters of the ex-core detector under different operating conditions and the corresponding reference power distribution parameters according to the comparison result based on the non-parametric statistical analysis method, that is, the computer device can quantify the indication signal of the ex-core detector based on the non-parametric statistical analysis method.

In the method for quantifying the indication signal of the nuclear instrument system provided in the embodiment of the present application, the power distributions of the reactor core under different operating conditions are simulated to obtain the power distribution parameters of the reactor core under the different operating conditions, and the indication signal of the ex-core detector is quantified based on the non-parametric statistical analysis method according to the power distribution parameters of the reactor core under different operating conditions and the corresponding reference power distribution parameters. This method considers the power distribution parameters of the reactor core under different operating conditions, and quantifies the indication signal of the ex-core detector based on the power distribution parameters of the reactor core under different operating conditions and the reference power distribution parameters. Compared with the prior art, this solution does not require the collection of measured data under actual operating conditions and is suitable for designing control and protection signal setpoints for a new reactor core where actual measurement data is scarce. Therefore, this solution has strong applicability. In addition, this solution quantifies the uncertainty of the indication signal of the ex-core detector through theoretical analysis, thereby obtaining a more accurate indication signal.

In an embodiment, based on the embodiment shown in FIG. 2, a process of simulating the power distributions of the reactor core under different operating conditions to obtain the power distribution parameters of the reactor core under different operating conditions may be described. As shown in FIG. 3, the step S201 of simulating the power distributions of the reactor core under different operating conditions to obtain the power distribution parameters of the reactor core under different operating conditions may include the following steps.

At S301, an actual physical model of the reactor core is constructed.

The actual physical model of the reactor core can be a reactor model, or can be the reactor core simulation model mentioned in the above embodiments. The actual physical model of the reactor core is used to determine the power distribution parameters of the reactor core under different operating conditions based on characteristic parameters under different operating conditions. Optionally, the actual physical model of the reactor core can be trained based on data samples of an actual reactor core under different operating conditions and a pre-constructed initial reactor core model.

In an embodiment of the present application, when the uncertainty of the indication signal of the ex-core detector needs to be evaluated, the computer device can construct the corresponding actual physical model of the reactor core based on geometric parameters or physical parameters of the reactor core. Optionally, the initial reactor core model can be pre-constructed, and then actual sample data of the reactor core under different operating conditions can be obtained, and the initial reactor core model can be trained based on these sample data to obtain the physical model of the reactor core.

At S302, characteristic parameters under the different operating conditions are obtained.

The categories of characteristic parameters under operating conditions can be classified into perturbations of different cycle burnups, different reactor core loading patterns, different control rod configurations, different power levels, or different xenon oscillation phases. Where, the perturbations of different burnups cover typical burnup stages from the beginning to the end of cycle. The different loading patterns include typical fuel management cycle patterns that may occur during operation of a power plant. The different control rod configurations include a control rod at the top, typical rod configurations in the optimal control range, and an insertion limit position. The different xenon oscillation phases include an instantaneous xenon, a xenon peak, an equilibrium xenon and other typical xenon characteristic conditions.

In an embodiment of the present application, a set of characteristic parameters for a specific operating condition can be obtained by extracting any single item from each of the aforementioned categories and combining them through permutation and combination. Furthermore, by traversing and extracting all possible conditions from each category for combination and subsequently screening the operating constraints, the characteristic parameters covering different operating conditions within the range of operating constraints can be obtained. Exemplarily, the perturbation of the BLX, the C01 cycle loading pattern, the R rod at the typical control rod position ARO, and the GN rod at the typical control rod position ARO are combined to obtain characteristic parameters under one operating condition. Furthermore, the perturbation at 6000MWd/tU, the C01 cycle loading pattern, the R rod at the typical control rod position ARO, and the GN rod at the typical control rod position ARO are combined to obtain characteristic parameters under another operating condition. All categories of the characteristic parameter are traversed to obtain the characteristic parameters under the different operating conditions, and then the operating constraints are screened to obtain characteristic parameters covering different operating conditions within the range of the operating constraints.

At S303, the characteristic parameters under different operating conditions are inputted into the actual physical model of the reactor core to obtain the power distribution parameters of the reactor core under different operating conditions.

In the embodiment of the present application, the computer device may input the characteristic parameters under different operating conditions obtained in the above step S302 into the constructed actual physical model of the reactor core, and perform calculations to obtain calculation results, which may characterize the power distribution parameters of the reactor core under different operating conditions.

Furthermore, the power distributions of the reactor core under the normal operating conditions and the power distributions of the reactor core under the accident operating conditions are simulated to obtain the power distribution parameters of the reactor core under the normal operating conditions and the power distribution parameters of the reactor core under the accident operating conditions.

Exemplarily, the power distributions of the reactor core under the normal operating conditions can be the characteristic parameters obtained by combining the perturbation of the BLX, the C01 cycle loading pattern, the R rod at the typical control rod position ARO, and the GN rod at the typical control rod position ARO. These parameters are input into the actual physical model of the reactor core for calculation to obtain the power distribution parameters of the reactor core under the normal operating conditions. Subsequently, the accident process is simulated based on the power distributions of the reactor core under the normal operating conditions to obtain the power distribution parameters of the reactor core under the accident operating conditions.

In the method of obtaining the power distribution parameters of the reactor core under the different operating conditions, which is provided in the embodiments of the present application, the power distributions under the normal operating conditions, under the accident operating conditions and during the calibration process are taken into account, and the power distribution parameters under the different operating conditions are obtained based on the power distributions under the different operating conditions, which makes the considered operating conditions more comprehensive, thereby enabling the subsequent quantification of the uncertainty of the indication signal of the ex-core detector to be more accurate.

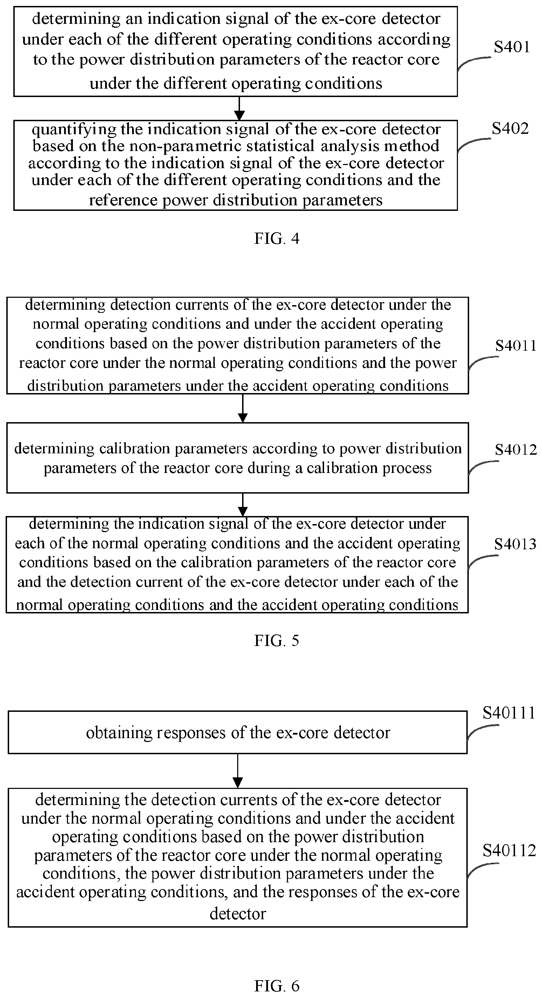

In an embodiment, based on the embodiment shown in FIG. 2, a process of quantifying the indication signal of the ex-core detector based on the non-parametric statistical analysis method according to the power distribution parameters of the reactor core under different operating conditions and the corresponding reference power distribution parameters can be described. As shown in FIG. 4, the step S202 of quantifying the indication signal of the ex-core detector based on the non-parametric statistical analysis method according to the power distribution parameters of the reactor core under the different operating conditions and the corresponding reference power distribution parameters can include the following steps.

At S401, an indication signal of the ex-core detector under each of the different operating conditions is determined according to the power distribution parameters of the reactor core under the different operating conditions.

The indication signal of the reactor core under the different operating conditions may include but is not limited to a nuclear power Pr and an axial power deviation signal $\Delta I$.

In an embodiment of the present application, after obtaining the power distribution parameters of the reactor core under the different operating conditions, the computer device performs calculations based on the obtained power distribution parameters of the reactor core under different operating conditions to obtain calculation results. The calculation results can characterize the indication signals of the ex-core detector under the different operating conditions. Exemplarily, the obtained power distribution parameters of the reactor core under the different operating conditions can be substituted into the preset physical model to perform numerical calculations to obtain the indication signals of the ex-core detector under the different operating conditions. In another exemplary embodiment, calculations can be performed based on the obtained power distribution parameters of the reactor core under the different operating conditions and other parameters of the reactor core to obtain the indication signals of the ex-core detector under the different operating conditions.

In an embodiment of the present application, the indication signals of the ex-core detector under the different operating conditions can be determined based on the power distribution parameters of the reactor core under the different operating conditions and the physical model of the reactor core. A method for determining the indication signals of the ex-core detector under the different operating conditions is provided hereinafter.

Exemplarily, as shown in FIG. 5, the different operating conditions include normal operating conditions and accident operating conditions. The step S401 of determining the indication signal of the ex-core detector under each of the different operating conditions according to the power distribution parameters of the reactor core under the different operating conditions includes:

At S4011, detection currents of the ex-core detector under the normal operating conditions and under the accident operating conditions are determined based on the power distribution parameters of the reactor core under the normal operating conditions and the power distribution parameters under the accident operating conditions.

Each detection current may be a response current of the detector.

In an embodiment of the present application, after obtaining the power distribution parameters of the reactor core under the normal operating conditions and under the accident operating conditions, the computer device performs calculations based on the obtained power distribution parameters of the reactor core under the normal operating conditions and under the accident operating conditions to obtain the calculation results. The calculation results can characterize the detection currents of the ex-core detector under the normal operating conditions and under the accident operating conditions, respectively. Exemplarily, the obtained power distribution parameters of the reactor core under the normal operating conditions and under the accident operating conditions can be substituted into a preset current model for numerical calculation to obtain the detection currents of the ex-core detector under the normal operating conditions and under the accident operating conditions, respectively. In another exemplary embodiment, the numerical calculations can be performed based on the obtained power distribution parameters of the reactor core under the normal operating conditions and under the accident operating conditions and other parameters of the reactor core to obtain the detection currents of the ex-core detector under the normal operating conditions and under the accident operating conditions.

In an embodiment of the present application, the detection currents of the ex-core detector under the normal operating conditions and under the accident operating conditions can be determined according to the power distribution parameters of the reactor core under different operating conditions and the response of the ex-core detector. A method for determining the detection currents of the ex-core detector under the normal operating conditions and under the accident operating conditions is provided below.

Exemplarily, as shown in FIG. 6, the step S4011 of determining the detection currents of the ex-core detector under the normal operating conditions and under the accident operating conditions based on the power distribution parameters of the reactor core under the normal operating conditions and the power distribution parameters under the accident operating conditions includes following steps.

At S40111, responses of the ex-core detector are obtained.

Where, the response of the ex-core detector can be a probability that a neutron originating in the core, after undergoing scattering collisions, reaches the ex-core detector. Generally, the response of the ex-core detector of a node at a position (x, y, z) in the reactor core can be expressed by $w(x, y, z)$.

In an embodiment of the application, the computer device can directly obtain the response of the ex-core detector from a preset model.

At S40112, the detection currents of the ex-core detector under the normal operating conditions and under the accident operating conditions are determined based on the power distribution parameters of the reactor core under the normal operating conditions, the power distribution parameters under the accident operating conditions, and the responses of the ex-core detector.

In an embodiment of the present application, a computer device may perform the numerical calculations on the power distribution parameters of the reactor core under the normal operating conditions and under the accident operating conditions and the responses of the ex-core detector, which are obtained in the above steps, to obtain the numerical calculation results. The numerical calculation results may characterize the detection currents of the ex-core detector under the normal operating conditions and under the accident operating conditions. Exemplarily, the detection current of the ex-core detector under each of the different operating conditions may be expressed by the following formula (1):

$$I = C \times \sum_{\substack{x=1,2,\cdots,n \\ y=1,2,\cdots,n \\ z=1,2,\cdots,n}} R(x, y, z) \qquad (1)$$

where, $I$ represent the detection current of the ex-core detector under each of the different operating conditions, and $C$ represents a coefficient related to a sensitivity of the ex-core detector. This coefficient is generally a constant. $R(x, y, z)$ represents a reaction rate generated in the detector by neutrons originating from fission in a node at any position $(x, y, z)$ in the reactor core. It should be noted that the detection current under each of the different operating conditions varies with changes in the reaction rate generated in the detector by neutrons originating from fission within the node.

Furthermore, in the formula (1), the reaction rate $R(x, y, z)$ generated in the detector by the neutrons originating from fission within the node at any position $(x, y, z)$ in the reactor core can be expressed by the following formula (2):

$$R(x, y, z) = w(x, y, z) \times \left[ v(x, y, z) \times \sum_f(x, y, z) \times \Phi(x, y, z) \right] \qquad (2)$$

where $w(x, y, z)$ represents the scattering probability of the neutron in the node at any position (x, y, z) in the reactor core, characterizes the probability that the neutron, after undergoing scattering collisions, reaches the ex-core detector, and can be obtained by simulation in a reactor model through the computer device. $[v(x, y, z) \times \Sigma_f(x, y, z) \times \Phi(x, y, z)]$ is the power distribution parameter of the reactor core under each of the normal operating conditions and accident operating conditions, characterizes the production rate of fission neutrons at any position (x, y, z) in the reactor core, and is related to the reactor core state and power distribution. Where $\Phi(x, y, z)$ can be calculated by using a deterministic program.

At S4012, calibration parameters are determined according to power distribution parameters of the reactor core during a calibration process.

The power distribution parameters of the reactor core during the calibration process may be the power distribution parameters of the ideal operation (normal operation) of the reactor core during a trial operation. The calibration parameters may be the indication signals ($Pr$ and $\Delta I$) measured in the reactor core under the calibration condition (under the full power condition) and a deviation of the signals ($Pr'$ and $\Delta I'$) calculated based on the responses of the ex-core detector.

The calibration coefficients of the detection current generally include: $\alpha$, a calibration coefficient $K_U$ of an upper-detector current and a calibration coefficient $K_L$ of a lower-detector current.

In an embodiment of the present application, the computer device may perform numerical calculations on the power distribution parameters of the reactor core during the calibration process determined in the above steps to obtain the calibration parameters. Exemplarily, the power distribution parameters of the reactor core during the calibration process determined in the above steps may be substituted into the above formulas (1) and (2) to obtain the detection current of the reactor core under the calibration condition, and then the numerical calculation may be performed on the detection current of the reactor core under the calibration condition to obtain the calibration parameters.

In the embodiment of the present application, the calibration parameters can be determined based on the power distribution parameters of the reactor core under different operating conditions and the scattering probability related with the reactor core. A method for determining the calibration parameters is provided below.

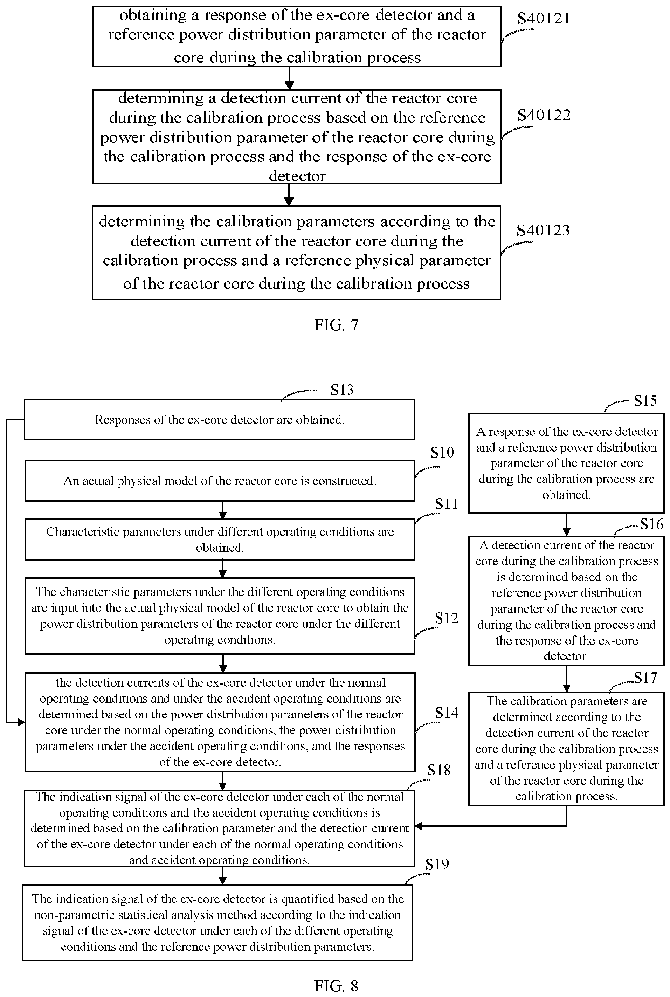

Exemplarily, as shown in FIG. 7, the step S4012 of determining the calibration parameters according to the power distribution parameters of the reactor core during the calibration process includes following steps.

At S40121, a response of the ex-core detector and a reference power distribution parameter of the reactor core during the calibration process are obtained.

In an embodiment of the present application, the computer device can directly obtain the response of the ex-core detector through a preset model, where the preset model can be a reactor model. Alternatively, the reference power distribution parameter of the reactor core during the calibration process, that is, the ideal power distribution parameter of the reactor core during the trial operation, which is preset in the computer device, can also be obtained.

At S40122, a detection current of the reactor core during the calibration process is determined based on the reference power distribution parameter of the reactor core during the calibration process and the response of the ex-core detector.

The detection current of the reactor core during the calibration process may be an ideal detection current of the reactor core during the trial operation process.

In an embodiment of the present application, a calculation can be performed on the reference power distribution parameter of the reactor core during the calibration process and the response of the ex-core detector to obtain a calculation result, which represents the detection current of the reactor core during the calibration process. Exemplarily, the reference power distribution parameter of the reactor core during the calibration process and the response of the ex-core detector during the calibration process can be substituted into the above formulas (1) and (2) to obtain the detection current of the reactor core during the calibration process.

At S40123, the calibration parameters are determined according to the detection current of the reactor core during the calibration process and a reference physical parameter of the reactor core during the calibration process.

In an embodiment of the present application, the computer device can first obtain an indication signal of the reactor core during the calibration process, which characterizes an ideal nuclear power and an axial power deviation parameter of the reactor core during the trial operation, and then determine the calibration parameters based on the detection current of the reactor core during the calibration process and the reference physical parameter of the reactor core during the calibration process.

Exemplarily, the calibration parameter (the nuclear thermal power) of the reactor core can be expressed by the following formula (3):

$$Pr = K_U \times I_U + K_L \times I_L \qquad (3)$$

where, $Pr$ represents the nuclear power of the reactor core, $K_U$, $K_L$ represent the calibration coefficients of the upper-detector current and the lower-detector current, respectively, $I_U$ and $I_L$ represent are the upper-detector current and the lower-detector current, respectively.

Exemplarily, the calibration parameter (the axial power deviation parameter) of the reactor core can be expressed by the following formula (4):

$$\Delta I = \alpha(K_U \times I_U - K_L \times I_L) \qquad (4)$$

where, $\Delta I$ represents the axial power deviation parameter of the reactor core, $K_U$, $K_L$ represent the calibration coefficients of the upper-detector current and the lower-detector current, respectively, $I_U$, $I_L$ represent the upper-detector current and the lower-detector current, respectively, and $\alpha$ represents a general calibration coefficient of the detection current.

At S4013, the indication signal of the ex-core detector under each of the normal operating conditions and the accident operating conditions is determined based on the calibration parameters and the detection current of the ex-core detector under each of the normal operating conditions and the accident operating conditions.

In the embodiment of the present application, after the calibration parameters and the detection current of the reactor core under each of the normal operating conditions and accident operating conditions are obtained, the calibration parameters and the detection current of the reactor core under each of the normal operating conditions and accident operating conditions can be substituted into a preset nuclear physics model to obtain the indication signal of the ex-core detector under each of the normal operating conditions and accident operating conditions. Exemplarily, the calibration parameters and the detection current of the reactor core under each of the normal operating conditions and accident operating conditions can be substituted into the above formulas (3) and (4) to obtain the indication signal of the ex-core detector under each of the normal operating conditions and accident operating conditions.

At S402, the indication signal of the ex-core detector is quantified based on the non-parametric statistical analysis method according to the indication signal of the ex-core detector under each of the different operating conditions and the reference power distribution parameters.

The reference power distribution parameters can be obtained through the reactor model of the deterministic program preset in a computer device, and the relationship model between the power distribution parameters and the detector responses can be obtained through a Monte Carlo program.

In the embodiment of the present application, the reference power distribution parameters of the reactor core under each of the different operating conditions can be substituted into the above formulas (1), (2), (3) and (4) respectively to obtain the reference indication signal of the reactor core under each of the different operating conditions. Then, the numerical calculations are performed on the indication signal of the ex-core detector under each of the different operating conditions and the reference indication signal of the reactor core under each the different operating conditions to obtain the numerical calculation result. The magnitude of the numerical calculation result characterizes the accuracy of the indication signal of the ex-core detector. Optionally, a difference between the indication signal of the ex-core detector under each of the different operating conditions and the reference indication signal of the reactor core under each of the different operating conditions can be first calculated, and then the ratio between the indication signal of the ex-core detector under each of the different operating conditions and this difference is calculated to obtain a first operation result. What's more, the difference between the indication signal of the ex-core detector under each of the different operating conditions and the reference indication signal of the reactor core under each of different operating conditions can be calculated, and then the ratio between the reference indication signal of the reactor core under each of the different operating conditions and the difference is calculated to obtain a second operation result. Then the first operation result is compared with the second operation result, and the smaller the obtained numerical value, the higher the accuracy of the indication signal of the ex-core detector, and the larger the obtained numerical value, the lower the accuracy of the indication signal of the ex-core detector.

An embodiment of the present application provides a method for quantifying the indication signal of the ex-core detector, that is, the uncertainty of the indication signal of the ex-core detector are statistically analyzed by using the WILKS non-parametric statistical analysis method. Exemplarily, the deviation $\{x_1, x_2, \cdots x_N\}$ between the indication signal $Pr\{Pr_1, PrI_2, \cdots Pr_N\}$ or $\Delta I\{\Delta I_1, \Delta I_2, \cdots \Delta I_N\}$ of the ex-core detector and the reference indication signal $Pr$ or $\Delta I$ of the reactor core is obtained by calculating a difference between the obtained indication signal of the ex-core detector and the reference indication signal of the reactor core. Where, $\{x_1, x_2, \cdots x_N\}$ can be considered as $N$ independent outputs of the random function $x$ related to the states of the reactor core, whose probability distribution functions $f(x)$ are continuous. Then $\{x_1, x_2, \cdots x_N\}$ are arranged in an ascending order to form a sampling result, and it is defined that $x_0 = -\infty$, and $x_{N+1} = +\infty$.

In the WILKS non-parametric statistical analysis method, for a probability share $\gamma$, a tolerance interval *(L, U)* can be determined by the Wilks non-parametric statistical, so that the confidence level $\beta$ of the probability $P(\int_L^U f(x)dx > \gamma)$ within the tolerance interval *(L, U)* is expressed by the following formula (5):

$$\beta = \sum_{j=0}^{s-r-1} \binom{N}{j} \gamma^j (1 - \gamma)^{N-j} \tag{5}$$

where $N$ represents the quantity of samples, r and s are orders representing lower and upper limits of the sample confidence interval respectively, $0 \leq r < s \leq N$, $L=y(r)$, $U=y(s)$, and $\binom{N}{j} = \frac{N!}{j! \times (N-j)!}$ .

If the axial power deviation parameter $\Delta I$ of the indication signal of the ex-core detector falls within the required range, it is considered that the reactor core operates safely and will not trigger a protection signal of the reactor protection system. Table 1 presents the number of calculations required to meet the confidence interval with a confidence level of 95%, as derived from Equation (5). If the quantity n of samples, the confidence level of 95% and the probability share of 95% of measurement deviations of the indication signal of the ex-core detector are given, the position $N(r)<n<N(r+1)$ corresponding to the sample quantity $n$ can be found in a right data column of Table 1. The order r for this group of sample points meeting two values 95% is determined according to Table 1, then the 95% confidence interval for this sample point is *(r+1, N-r-1)*. This interval shows that, after considering the 95% confidence interval, the uncertainty range of the indication signal of the ex-core detector is *(r+1, N-r-1)*. Exemplarily, in an analysis of a typical cycle of a third-generation nuclear power unit, the maximum positive deviation of the nuclear power of the indication signal was 5.08%, and the maximum negative deviation thereof was -1.87%. The maximum positive deviation of the axial power deviation signal was 2.64%, and the maximum negative deviation thereof was -1.23%. After considering the confidence interval of 95%, the uncertainty range of the nuclear power is (-1.05, 3.85), and the uncertainty range of the axial power deviation signal is (-0.94, 1.97).

Table 1 Number of Calculations Required to Meet Confidence Interval with 95% Confidence Level

| Order *r* satisfying two values 95% | Required Number *N* of Calculations |
| --- | --- |
| 1 | 59 |
| 2 | 93 |
| 3 | 124 |
| 4 | 153 |
| 5 | 181 |
| 6 | 208 |
| 7 | 234 |
| 8 | 260 |

(continued)

| Order *r* satisfying two values 95% | Required Number *N* of Calculations |
| --- | --- |
| 9 | 286 |
| 10 | 311 |
| 11 | 336 |
| 12 | 361 |
| 13 | 386 |
| 14 | 410 |
| 15 | 434 |
| 16 | 458 |
| 17 | 482 |
| 18 | 506 |
| 19 | 530 |
| 20 | 554 |
| N/20 | very large |

In the method for quantifying the indication signal of the nuclear instrument system in the embodiment of the present application, the power distribution parameters of the reactor core under different operating conditions are taken into account, and the indication signal of the ex-core detector is quantified based on the power distribution parameters of the reactor core under different operating conditions and the reference power distribution parameters. Compared with the prior art, this solution does not require the collection of measured data under actual operating conditions, and can quantify the uncertainty of the indication signal of the ex-core detector through theoretical analysis, thereby being suitable for designing control and protection signal setpoints for a new reactor core where actual measurement data is scarce. In addition, this method adopts the WILKS non-parametric distribution statistical method, has no requirements for the overall distribution type of the data, and does not rely on the deviation between the indication signal and the reference indication signal. The confidence interval can be determined based on the quantity of the samples and the confidence level, thereby making the determined uncertainty of the indication signal of the ex-core detector more accurate.

FIG. 8 is a flow chart of the method for quantifying the indication signal of the nuclear instrument system provided in an embodiment of the present application. Summarizing the methods described in all embodiments above, as shown in FIG. 8, the method may include the following steps.

At S10, an actual physical model of the reactor core is constructed.

At S11, characteristic parameters under different operating conditions are obtained.

At S12, the characteristic parameters under different operating conditions are inputted into the actual physical model of the reactor core to obtain the power distribution parameters of the reactor core under different operating conditions.

At S13, responses of the ex-core detector are obtained.

At S14, the detection currents of the ex-core detector under the normal operating conditions and under the accident operating conditions are determined based on the power distribution parameters of the reactor core under the normal operating conditions, the power distribution parameters under the accident operating conditions, and the responses of the ex-core detector.

At S15, a response of the ex-core detector and a reference power distribution parameter of the reactor core during the calibration process are obtained.

At S16, a detection current of the reactor core during the calibration process is determined based on the reference power distribution parameter of the reactor core during the calibration process and the response of the ex-core detector.

At S17, the calibration parameters are determined according to the detection current of the reactor core during the calibration process and a reference physical parameter of the reactor core during the calibration process.

At S18, the indication signal of the ex-core detector under each of the normal operating conditions and the accident operating conditions is determined based on the calibration parameter and the detection current of the ex-core detector under each of the normal operating conditions and the accident operating conditions.

At S19, the indication signal of the ex-core detector is quantified based on the non-parametric statistical analysis method according to the indication signal of the ex-core detector under each of the different operating conditions and the reference power distribution parameters.

In the method for quantifying the indication signal of the nuclear instrument system provided in the embodiment of the present application, the power distributions of the reactor core under different operating conditions are simulated to obtain the power distribution parameters of the reactor core under the different operating conditions, and the indication signal of the ex-core detector is quantified based on the non-parametric statistical analysis method according to the power distribution parameters of the reactor core under different operating conditions and the corresponding reference power distribution parameters. This method considers the power distribution parameters of the reactor core under different operating conditions, and quantifies the indication signal of the ex-core detector based on the power distribution parameters of the reactor core under different operating conditions and the reference power distribution parameters. Compared with the prior art, this solution does not require the collection of measured data under actual operating conditions and is suitable for designing control and protection signal setpoints for a new reactor core where actual measurement data is scarce. Therefore, this solution has strong applicability. In addition, this solution quantifies the uncertainty of the indication signal of the ex-core detector through theoretical analysis, thereby obtaining a more accurate indication signal.

It should be understood that, although the steps in the flowcharts involved in the embodiments described above are displayed sequentially as indicated by the arrows, these steps are not necessarily executed sequentially in the order indicated by the arrows. Unless otherwise specified herein, there is no strict order limitation for the execution of these steps, and these steps may be executed in other orders. Moreover, at least a part of the steps in the flowcharts involved in the embodiments described above may include multiple steps or multiple stages. These steps or stages are not necessarily executed at the same time, but may be executed at different times. The execution order of these steps or stages is not necessarily sequential, but may be executed in turn or alternately with other steps or at least part of the steps or stages in other steps.

Based on the same inventive concept, embodiments of the present application further provide an apparatus for quantifying a signal, which is configured to implement the method for quantifying the indication signal of the nuclear instrument system above. The solution provided by the apparatus is similar to the solution described in the aforementioned method. Therefore, the specific definitions of the apparatus for quantifying the signal provided one or more embodiments below can be found in the aforementioned definitions of the method for quantifying the indication signal of the nuclear instrument system, which are not described repeatedly herein.

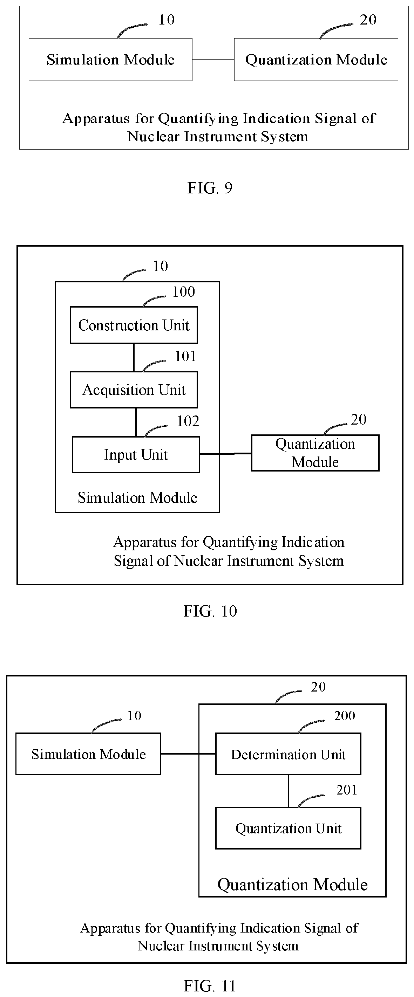

In an embodiment, as shown in FIG. 9, an apparatus for quantifying an indication signal of a nuclear instrument system, including a simulation module 10 and a quantification module 20, is provided.

The simulation module 10 is configured to simulate power distributions of a reactor core under different operating conditions to obtain power distribution parameters of the reactor core under the different operating conditions.

The quantization module 20 is configured to quantify an indication signal of an ex-core detector based on a non-parametric statistical analysis method according to the power distribution parameters of the reactor core under different operating conditions and corresponding reference power distribution parameters.

In an embodiment, the simulation module 10 includes a construction unit 100, an acquisition unit 101, and an input unit 102.

The construction unit 100 is specifically configured to construct an actual physical model of the reactor core.

The acquisition unit 101 is specifically configured to obtain characteristic parameters under the different operating conditions.

The input unit 102 is specifically configured to input the characteristic parameters under different operating conditions into the actual physical model of the reactor core to obtain the power distribution parameters of the reactor core under different operating conditions.

In an embodiment, the different operating conditions include normal operating conditions and accident operating conditions. The simulation module 10 can simulate the power distributions of the reactor core under the normal operating conditions and the power distributions of the reactor core under the accident operating conditions to obtain the power distribution parameters of the reactor core under the normal operating conditions and the power distribution parameters of the reactor core under the accident operating conditions.

In an embodiment, as shown in FIG. 11, the quantization module 20 includes a determination unit 200 and a quantization unit 201.

The determination unit 200 is specifically configured to determine indication signals of the ex-core detector under the different operating conditions according to the power distribution parameters of the reactor core under the different operating conditions.

The quantization unit 201 is specifically configured to quantify the indication signal of the ex-core detector based on the non-parametric statistical analysis method according to the indication signal of the ex-core detector under each of the different operating conditions and the reference power distribution parameters.

In an embodiment, the different operating conditions include normal operating conditions and accident operating conditions. The determination unit 200 is specifically configured to determine detection currents of the ex-core detector under the normal operating conditions and under the accident operating conditions based on the power distribution parameters of the reactor core under the normal operating conditions and the power distribution parameters under the accident operating conditions; determine calibration parameters according to power distribution parameters of the reactor core during a calibration process; and determine the indication signal of the ex-core detector under each of the normal operating conditions and accident operating conditions based on the calibration parameters and the detection current of the ex-core detector under each of the normal operating conditions and accident operating conditions.

In an embodiment, the determination unit 200 is specifically configured to obtain responses of the ex-core detector; and determine the detection currents of the ex-core detector under the normal operating conditions and under the accident operating conditions based on the power distribution parameters of the reactor core under the normal operating conditions, the power distribution parameters under the accident operating conditions, and the responses of the ex-core detector.

In an embodiment, the determination unit 200 is specifically configured to obtain a response of the ex-core detector and a reference power distribution parameter of the reactor core during the calibration process; determine a detection current of the reactor core during the calibration process based on the reference power distribution parameter of the reactor core during the calibration process and the response of the ex-core detector; and determine calibration parameters according to the detection current of the reactor core during the calibration process and a reference physical parameter of the reactor core during the calibration process.

Each module in the apparatus for quantifying the indication signal of the nuclear instrument system may be fully or partially implemented by software, hardware or a combination thereof. The above modules may be embedded in or independent of a processor in a computer device in the form of hardware, or may be stored in a memory in a computer device in the form of software, so that the processor may call and execute operations corresponding to the above modules.

In an embodiment, a computer device is provided, which may be a server, and an internal structure thereof may be as shown in FIG. 1. The computer device includes a processor, a memory, an input/output (I/O) interface, and a communication interface. The processor, the memory, and the I/O interface are connected via a system bus, and the communication interface is connected to the system bus via the I/O interface. The processor of the computer device provides computing and control capabilities. The memory of the computer device includes a non-transitory storage medium and an internal memory. The non-transitory storage medium stores an operating system, a computer program, and a database. The internal memory provides an environment for the operation of the operating system and the computer program in the non-transitory storage medium. The database of the computer device stores indication signal data. The I/O interface of the computer device is used to exchange information between the processor and external devices. The communication interface of the computer device is used to communicate with external terminals via a network connection. The computer program, when executed by the processor, implements the method for quantifying the indication signal of the nuclear instrument system.

Those skilled in the art may understand that the structure shown in FIG. 1 is merely a block diagram of a partial structure related to the solution of the present application, and does not constitute a limitation on the computer device to which the solution of the present application is applied. The specific computer device may include more or fewer components than shown in the figure, or combine certain components, or have a different arrangement of components.

In an embodiment, a computer device is provided, including a memory and a processor. A computer program is stored in the memory, and the processor, when executing the computer program, performs the following steps:

simulating power distributions of a reactor core under different operating conditions to obtain power distribution parameters of the reactor core under the different operating conditions; and

quantifying the indication signal of an ex-core detector based on a non-parametric statistical analysis method according to the power distribution parameters of the reactor core under the different operating conditions and corresponding reference power distribution parameters.

In an embodiment, the processor, when executing the computer program, further performs the following steps:

constructing an actual physical model of the reactor core;

obtaining characteristic parameters under the different operating conditions; and

inputting the characteristic parameters under different operating conditions into the actual physical model of the reactor core to obtain the power distribution parameters of the reactor core under different operating conditions.

In an embodiment, the processor, when executing the computer program, further performs the following step: simulating the power distributions of the reactor core under the normal operating conditions and the power distributions of the reactor core under the accident operating conditions to obtain the power distribution parameters of the reactor core under the normal operating conditions and the power distribution parameters of the reactor core under the accident operating conditions.

In an embodiment, the processor, when executing the computer program, further performs the following steps:

determining an indication signal of the ex-core detector under each of the different operating conditions according to the power distribution parameters of the reactor core under the different operating conditions; and

quantifying the indication signal of the ex-core detector based on the non-parametric statistical analysis method according to the indication signal of the ex-core detector under each of the different operating conditions and the reference power distribution parameters.

In an embodiment, the processor, when executing the computer program, further performs the following steps:

determining detection currents of the ex-core detector under the normal operating conditions and under the accident operating conditions based on the power distribution parameters of the reactor core under the normal operating conditions and the power distribution parameters under the accident operating conditions;
determining calibration parameters according to power distribution parameters of the reactor core during a calibration process; and
determining the indication signal of the ex-core detector under each of the normal operating conditions and the accident operating conditions based on the calibration parameters of the reactor core and the detection current of the ex-core detector under each of the normal operating conditions and the accident operating conditions.

In an embodiment, the processor, when executing the computer program, further performs the following steps:

obtaining responses of the ex-core detector; and
determining the detection currents of the ex-core detector under the normal operating conditions and under the accident operating conditions based on the power distribution parameters of the reactor core under the normal operating conditions, the power distribution parameters under the accident operating conditions, and the responses of the ex-core detector.

In an embodiment, the processor, when executing the computer program, further performs the following steps:

obtaining a response of the ex-core detector and a reference power distribution parameter of the reactor core during the calibration process;
determining a detection current of the reactor core during the calibration process based on the reference power distribution parameter of the reactor core during the calibration process and the response of the ex-core detector; and
determining the calibration parameters according to the detection current of the reactor core during the calibration process and a reference physical parameter of the reactor core during the calibration process.

In an embodiment, a computer-readable storage medium is provided, and has a computer program is stored thereon. The computer program, when executed by a processor, performs the following steps:

simulating power distributions of a reactor core under different operating conditions to obtain power distribution parameters of the reactor core under the different operating conditions; and
quantifying the indication signal of an ex-core detector based on a non-parametric statistical analysis method according to the power distribution parameters of the reactor core under the different operating conditions and corresponding reference power distribution parameters.

In an embodiment, the computer program, when executed by a processor, further performs the following steps:

constructing an actual physical model of the reactor core;
obtaining characteristic parameters under the different operating conditions; and
inputting the characteristic parameters under different operating conditions into the actual physical model of the reactor core to obtain the power distribution parameters of the reactor core under different operating conditions.

In an embodiment, the computer program, when executed by a processor, further performs the following steps:
simulating the power distributions of the reactor core under the normal operating conditions and the power distributions of the reactor core under the accident operating conditions to obtain the power distribution parameters of the reactor core under the normal operating conditions and the power distribution parameters of the reactor core under the accident operating conditions.
In an embodiment, the computer program, when executed by a processor, further performs the following steps:

determining an indication signal of the ex-core detector under each of the different operating conditions according to the power distribution parameters of the reactor core under the different operating conditions; and
quantifying the indication signal of the ex-core detector based on the non-parametric statistical analysis method according to the indication signal of the ex-core detector under each of the different operating conditions and the reference power distribution parameters.

In an embodiment, the computer program, when executed by a processor, further performs the following steps:

determining detection currents of the ex-core detector under the normal operating conditions and under the accident operating conditions based on the power distribution parameters of the reactor core under the normal operating conditions and the power distribution parameters under the accident operating conditions;
determining calibration parameters according to power distribution parameters of the reactor core during a calibration process; and
determining the indication signal of the ex-core detector under each of the normal operating conditions and the accident operating conditions based on the calibration parameters of the reactor core and the detection current of the ex-core detector under each of the normal operating conditions and the accident operating conditions.

In an embodiment, the computer program, when executed by a processor, further performs the following steps:

obtaining responses of the ex-core detector; and
determining the detection currents of the ex-core detector under the normal operating conditions and under the accident operating conditions based on the power distribution parameters of the reactor core under the normal operating conditions, the power distribution parameters under the accident operating conditions, and the responses of the ex-core detector.

In an embodiment, the computer program, when executed by a processor, further performs the following steps:

obtaining a response of the ex-core detector and a reference power distribution parameter of the reactor core during the calibration process;
determining a detection current of the reactor core during the calibration process based on the reference power distribution parameter of the reactor core during the calibration process and the response of the ex-core detector; and
determining the calibration parameters according to the detection current of the reactor core during the calibration process and a reference physical parameter of the reactor core during the calibration process.

In an embodiment, a computer program product is provided, comprising a computer program. The computer program, when executed by a processor, causes the processor to perform the following steps:

simulating power distributions of a reactor core under different operating conditions to obtain power distribution parameters of the reactor core under the different operating conditions; and
quantifying the indication signal of an ex-core detector based on a non-parametric statistical analysis method according to the power distribution parameters of the reactor core under the different operating conditions and corresponding reference power distribution parameters.

In an embodiment, the computer program, when executed by a processor, causes the processor to further perform the following steps:

constructing an actual physical model of the reactor core;
obtaining characteristic parameters under the different operating conditions; and
inputting the characteristic parameters under different operating conditions into the actual physical model of the reactor core to obtain the power distribution parameters of the reactor core under different operating conditions.

In an embodiment, the computer program, when executed by a processor, causes the processor to further perform the following steps:
simulating the power distributions of the reactor core under the normal operating conditions and the power distributions of the reactor core under the accident operating conditions to obtain the power distribution parameters of the reactor core under the normal operating conditions and the power distribution parameters of the reactor core under the accident operating conditions.

In an embodiment, the computer program, when executed by a processor, causes the processor to further perform the following steps:

determining an indication signal of the ex-core detector under each of the different operating conditions according to the power distribution parameters of the reactor core under the different operating conditions; and
quantifying the indication signal of the ex-core detector based on the non-parametric statistical analysis method according to the indication signal of the ex-core detector under each of the different operating conditions and the reference power distribution parameters.

In an embodiment, the computer program, when executed by a processor, causes the processor to further perform the following steps:

determining detection currents of the ex-core detector under the normal operating conditions and under the accident operating conditions based on the power distribution parameters of the reactor core under the normal operating conditions and the power distribution parameters under the accident operating conditions;
determining calibration parameters according to power distribution parameters of the reactor core during a calibration process; and
determining the indication signal of the ex-core detector under each of the normal operating conditions and the accident operating conditions based on the calibration parameters of the reactor core and the detection current of the ex-core detector under each of the normal operating conditions and the accident operating conditions.

In an embodiment, the computer program, when executed by a processor, causes the processor to further perform the following steps:

obtaining responses of the ex-core detector; and
determining the detection currents of the ex-core detector under the normal operating conditions and under the accident operating conditions based on the power distribution parameters of the reactor core under the normal operating conditions, the power distribution parameters under the accident operating conditions, and the responses of the ex-core detector.

In an embodiment, the computer program, when executed by a processor, causes the processor to further perform the following steps:

obtaining a response of the ex-core detector and a reference power distribution parameter of the reactor core during the calibration process;
determining a detection current of the reactor core during the calibration process based on the reference power distribution parameter of the reactor core during the calibration process and the response of the ex-core detector; and
determining the calibration parameters according to the detection current of the reactor core during the calibration process and a reference physical parameter of the reactor core during the calibration process.

Those ordinary skilled in the art may understand that all or part of the process in the method of the above embodiments may be implemented by instructing the relevant hardware through a computer program, and the computer program may be stored in a non-transitory computer-readable storage medium. The computer program, when executed, may include the processes of the method embodiments above. Any reference made to the memory, the database, or other medium used in the various embodiments provided in this application may include at least one of non-transitory and transitory memory. Non-transitory memory may include read-only memory (ROM), magnetic tape, floppy disk, flash memory, optical memory, high-density embedded non-transitory memory, resistance random access memory (ReRAM), magneto resistive random-access memory (MRAM), ferroelectric random-access memory (FRAM), phase change memory (PCM), or graphene memory, and the like. The transitory memory may include random access memory (RAM) or external cache memory. As illustration but not limitation, the RAM may be in various forms, such as static random-access memory (SRAM) or dynamic random-access memory (DRAM), etc. The databases involved in the embodiments provided in the present application may include at least one of a relational database and a non-relational database. The non-relational databases may include, but are not limited to, a blockchain-based distributed database, and the like. The processors involved in the various embodiments provided herein may be, but are not limited to, a general-purpose processor, a central processing unit, a graphics processing unit, a digital signal processor, a programmable logic device, a quantum-computing-based data processing logic, and the like.

The technical features of the embodiments above may be combined arbitrarily. To make the description concise, not all possible combinations of the technical features in the above embodiments are described. However, as long as there are no contradictions in the combinations of these technical features, all of the combinations should be considered to be within the scope of the specification.

The embodiments above only represent several implementation modes of the present application, and the description thereof is relatively specific and detailed, but it should not be construed as limiting the scope of the present application. It should be noted that for those ordinary skilled in the art, various modifications and improvements may be made without departing from the concept of the present application, and all these modifications and improvements are within the protection scope of the present application. Therefore, the scope of protection of the present application should be subject to the appended claims.

**Claims**

1. A method for quantifying an indication signal of a nuclear instrument system, **characterized by** comprising:

   simulating power distributions of a reactor core under different operating conditions to obtain power distribution parameters of the reactor core under the different operating conditions; and
   quantifying the indication signal of an ex-core detector based on a non-parametric statistical analysis method according to the power distribution parameters of the reactor core under the different operating conditions and corresponding reference power distribution parameters.

2. The method according to claim 1, wherein simulating the power distributions of the reactor core under the different operating conditions to obtain the power distribution parameters of the reactor core under the different operating conditions comprises:

   constructing an actual physical model of the reactor core;
   obtaining characteristic parameters under the different operating conditions; and
   inputting the characteristic parameters under the different operating conditions into the actual physical model of the reactor core to obtain the power distribution parameters of the reactor core under the different operating conditions.

3. The method according to claim 1 or 2, wherein the different operating conditions comprise normal operating conditions and accident operating conditions, and simulating the power distributions of the reactor core under the different operating conditions to obtain the power distribution parameters of the reactor core under the different operating conditions comprises:
   simulating the power distributions of the reactor core under the normal operating conditions and the power distributions of the reactor core under the accident operating conditions to obtain the power distribution parameters of the reactor core under the normal operating conditions and the power distribution parameters of the reactor core under the accident operating conditions.

4. The method according to claim 1, wherein quantifying the indication signal of the ex-core detector based on the non-parametric statistical analysis method according to the power distribution parameters of the reactor core under the different operating conditions and the corresponding reference power distribution parameters comprises:

   determining the indication signal of the ex-core detector under each of the different operating conditions according to the power distribution parameters of the reactor core under the different operating conditions; and
   quantifying the indication signal of the ex-core detector based on the non-parametric statistical analysis method according to the indication signal of the ex-core detector under each of the different operating conditions and the reference power distribution parameters.

5. The method according to claim 4, wherein the different operating conditions comprise normal operating conditions and accident operating conditions, and determining the indication signal of the ex-core detector under each of the different operating conditions according to the power distribution parameters of the reactor core under the different operating conditions comprises:

   determining detection currents of the ex-core detector under the normal operating conditions and under the accident operating conditions based on the power distribution parameters of the reactor core under the normal operating conditions and the power distribution parameters under the accident operating conditions;
   determining calibration parameters according to power distribution parameters of the reactor core during a calibration process; and
   determining the indication signal of the ex-core detector under each of the normal operating conditions and the accident operating conditions based on the calibration parameters and the detection current of the ex-core detector under each of the normal operating conditions and the accident operating conditions.

6. The method according to claim 5, wherein determining the detection currents of the ex-core detector under the normal operating conditions and under the accident operating conditions based on the power distribution parameters of the reactor core under the normal operating conditions and the power distribution parameters under the accident operating conditions comprises:

    obtaining responses of the ex-core detector; and
    determining the detection currents of the ex-core detector under the normal operating conditions and under the accident operating conditions based on the power distribution parameters of the reactor core under the normal operating conditions, the power distribution parameters under the accident operating conditions, and the responses of the ex-core detector.

7. The method according to claim 5, wherein determining the calibration parameters according to the power distribution parameters of the reactor core during the calibration process comprises:

    obtaining a response of the ex-core detector and a reference power distribution parameter of the reactor core during the calibration process;
    determining a detection current of the reactor core during the calibration process based on the reference power distribution parameter of the reactor core during the calibration process and the response of the ex-core detector; and
    determining the calibration parameters according to the detection current of the reactor core during the calibration process and a reference physical parameter of the reactor core during the calibration process.

8. An apparatus for quantifying an indication signal of a nuclear instrument system, **characterized by** comprising:

    an acquisition module, configured to simulate power distributions of a reactor core under different operating conditions to obtain power distribution parameters of the reactor core under the different operating conditions; and
    a quantization module, configured to quantify the indication signal of an ex-core detector based on a non-parametric statistical analysis method according to the power distribution parameters of the reactor core under the different operating conditions and corresponding reference power distribution parameters.

9. A computer device, comprising a memory and a processor, wherein the memory has a computer program stored thereon, and the processor, when executing the computer program, performs steps of the method according to any one of claims 1 to 7.

10. A computer-readable storage medium, having a computer program stored thereon, wherein the computer program, when executed by a processor, performs steps of the method according to any one of claims 1 to 7.

Memory

Operating System

Computer Program

Database

| Processor | Internal Memory | Non-Transitory Storage Medium |

System Bus

I/O Interface

Communication Interface

Computer Device

FIG. 1

S201
simulating power distributions of a reactor core under different operating conditions to obtain power distribution parameters of the reactor core under the different operating conditions

S202
quantifying the indication signal of an ex-core detector based on a non-parametric statistical analysis method according to the power distribution parameters of the reactor core under the different operating conditions and corresponding reference power distribution parameters

FIG. 2

S301
constructing an actual physical model of the reactor core

S302
obtaining characteristic parameters under the different operating conditions

S303
inputting the characteristic parameters under different operating conditions into the actual physical model of the reactor core to obtain the power distribution parameters of the reactor core under the different operating conditions

FIG. 3

determining an indication signal of the ex-core detector under each of the different operating conditions according to the power distribution parameters of the reactor core under the different operating conditions ⟶ S401

quantifying the indication signal of the ex-core detector based on the non-parametric statistical analysis method according to the indication signal of the ex-core detector under each of the different operating conditions and the reference power distribution parameters ⟶ S402

FIG. 4

determining detection currents of the ex-core detector under the normal operating conditions and under the accident operating conditions based on the power distribution parameters of the reactor core under the normal operating conditions and the power distribution parameters under the accident operating conditions ⟶ S4011

determining calibration parameters according to power distribution parameters of the reactor core during a calibration process ⟶ S4012

determining the indication signal of the ex-core detector under each of the normal operating conditions and the accident operating conditions based on the calibration parameters of the reactor core and the detection current of the ex-core detector under each of the normal operating conditions and the accident operating conditions ⟶ S4013

FIG. 5

obtaining responses of the ex-core detector ⟶ S40111

determining the detection currents of the ex-core detector under the normal operating conditions and under the accident operating conditions based on the power distribution parameters of the reactor core under the normal operating conditions, the power distribution parameters under the accident operating conditions, and the responses of the ex-core detector ⟶ S40112

FIG. 6

| obtaining a response of the ex-core detector and a reference power distribution parameter of the reactor core during the calibration process | S40121 |

↓

| determining a detection current of the reactor core during the calibration process based on the reference power distribution parameter of the reactor core during the calibration process and the response of the ex-core detector | S40122 |

↓

| determining the calibration parameters according to the detection current of the reactor core during the calibration process and a reference physical parameter of the reactor core during the calibration process | S40123 |

FIG. 7

S13

| Responses of the ex-core detector are obtained. |

S10

| An actual physical model of the reactor core is constructed. |

S11

| Characteristic parameters under different operating conditions are obtained. |

↓

| The characteristic parameters under the different operating conditions are input into the actual physical model of the reactor core to obtain the power distribution parameters of the reactor core under the different operating conditions. | S12

↓

| the detection currents of the ex-core detector under the normal operating conditions and under the accident operating conditions are determined based on the power distribution parameters of the reactor core under the normal operating conditions, the power distribution parameters under the accident operating conditions, and the responses of the ex-core detector. | S14

↓

S18

| The indication signal of the ex-core detector under each of the normal operating conditions and the accident operating conditions is determined based on the calibration parameter and the detection current of the ex-core detector under each of the normal operating conditions and accident operating conditions. |

↓

S19

| The indication signal of the ex-core detector is quantified based on the non-parametric statistical analysis method according to the indication signal of the ex-core detector under each of the different operating conditions and the reference power distribution parameters. |

S15

| A response of the ex-core detector and a reference power distribution parameter of the reactor core during the calibration process are obtained. |

↓ S16

| A detection current of the reactor core during the calibration process is determined based on the reference power distribution parameter of the reactor core during the calibration process and the response of the ex-core detector. |

↓ S17

| The calibration parameters are determined according to the detection current of the reactor core during the calibration process and a reference physical parameter of the reactor core during the calibration process. |

FIG. 8

FIG. 9

FIG. 10

FIG. 11

## INTERNATIONAL SEARCH REPORT

| | International application No. |
|---|---|
| | **PCT/CN2024/080150** |

**A. CLASSIFICATION OF SUBJECT MATTER**

G06F 30/20(2020.01)i; G21C 17/00(2006.01)i

According to International Patent Classification (IPC) or to both national classification and IPC

**B. FIELDS SEARCHED**

Minimum documentation searched (classification system followed by classification symbols)

IPC: G06F,G21C

Documentation searched other than minimum documentation to the extent that such documents are included in the fields searched

Electronic data base consulted during the international search (name of data base and, where practicable, search terms used)

VEN, CNABS, CNTXT, WOTXT, EPTXT, USTXT, CNKI, IEEE: 堆芯, 参数, 分布, 评估, 量化, 功率, 非参数统计分析, react +, core, parameter, distribution, evaluation, quantization, power, nonparametric 1w statistics

**C. DOCUMENTS CONSIDERED TO BE RELEVANT**

| Category* | Citation of document, with indication, where appropriate, of the relevant passages | Relevant to claim No. |
|---|---|---|
| PX | CN 116467849 A (CHINA GENERAL NUCLEAR POWER CO., LTD. et al.) 21 July 2023 (2023-07-21) claims 1-10 | 1-10 |
| X | CN 114266157 A (NUCLEAR POWER INSTITUTE OF CHINA) 01 April 2022 (2022-04-01) description, paragraphs [0046]-[0073] | 1-10 |
| A | CN 109840353 A (CHINA AUTOMOTIVE BATTERY RESEARCH INSTITUTE CO., LTD.) 04 June 2019 (2019-06-04) entire document | 1-10 |
| A | US 6845342 B1 (THE UNITED STATES OF AMERICA AS REPRESENTED BY THE DEPARTMENT OF HEALTH AND HUMAN SERVICES) 18 January 2005 (2005-01-18) entire document | 1-10 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **28 May 2024** | **31 May 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

INTERNATIONAL SEARCH REPORT
Information on patent family members

International application No.

**PCT/CN2024/080150**

| Patent document cited in search report | | | Publication date (day/month/year) | Patent family member(s) | | | Publication date (day/month/year) |
|---|---|---|---|---|---|---|---|
| CN | 116467849 | A | 21 July 2023 | None | | | |
| CN | 114266157 | A | 01 April 2022 | None | | | |
| CN | 109840353 | A | 04 June 2019 | None | | | |
| US | 6845342 | B1 | 18 January 2005 | WO | 0072038 | A1 | 30 November 2000 |
| | | | | AU | 5160300 | A | 12 December 2000 |
| | | | | EP | 1204880 | A1 | 15 May 2002 |
| | | | | JP | 2003500137 | A | 07 January 2003 |
| | | | | CA | 2373536 | C | 10 February 2009 |
| | | | | JP | 2009056340 | A | 19 March 2009 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2023102832285